# EUROPÄISCHE PATENTSCHRIFT

(11) **EP 2 598 872 B1**
(45) Veröffentlichungstag und Bekanntmachung des Hinweises auf die Patenterteilung: **06.07.2016**
(21) Anmeldenummer: 11723399.9
(22) Anmeldetag: 19.05.2011
(51) Int. Cl.: G01N 30/08, G01N 30/20, G01N 30/40, G01N 30/46, G01N 1/40

(54) **VERFAHREN BASIEREND AUF EINE ANORDNUNG VON HILIC-CHROMATOGRAPHIESÄULE UND SPE-ANREICHERUNGSANORDNUNG ZUR PROBENAUFBEREITUNG UND ANALYSE VON PESTIZIDEN**
USE OF HILIC-CHROMATOGRAPHIC SEPARATION AND SOLID PHASE EXTRACTION FOR PREPARING AND ANALYZING PESTICIDES
MÉTHODE AVEC UTILISATION D'UNE COLONNE HILIC ET EXTRACTION SPE POUR LA PREPARATION ET L'ANALYSE DE PESTICIDES

(30) Priorität: 30.07.2010 DE 102010036770
(43) Veröffentlichungstag der Anmeldung: 05.06.2013
(73) Patentinhaber: joint analytical systems GmbH, 47445 Moers (DE)
(72) Erfinder: KITTLAUS, Stefan, 01277 Dresden (DE); RADTKE, Joerg, 47506 Neukirchen-Vluyn (DE)
(74) Vertreter: Weisse, Renate
(86) Internationale Anmeldenummer: PCT/EP2011/058145
(87) Internationale Veröffentlichungsnummer: WO 2012/013380

(56) Entgegenhaltungen:
- US-A1- 2010 024 527
- ALVAREZ-SANCHEZ B ET AL: "Automated determination of folate catabolites in human biofluids (urine, breast milk and serum) by on-line SPE-HILIC-MS/MS", JOURNAL OF CHROMATOGRAPHY, ELSEVIER SCIENCE PUBLISHERS B.V, NL, Bd. 1217, Nr. 28, 9. Juli 2010 (2010-07-09), Seiten 4688-4695, XP027085303, ISSN: 0021-9673 [gefunden am 2010-06-13]
- B INGELSE ET AL: "Determination of polar organophosphorus pesticides in aqueous samples by direct injection using liquid chromatography-tandem mass spectrometry", JOURNAL OF CHROMATOGRAPHY A, Bd. 918, Nr. 1, 18. Mai 2001 (2001-05-18), Seiten 67-78, XP55002643, ISSN: 0021-9673, DOI: 10.1016/S0021-9673(01)00660-4

## Beschreibung

### Technisches Gebiet

Die Erfindung betrifft ein Verfahren zur Probenaufbereitung und Analyse von Pestiziden in Proben mittels Chromatographie. Pestizide werden zum Schutz von Pflanzen gegen Schädlinge eingesetzt. Da diese als Rückstände in die Nahrungskette gelangen können, werden gesetzliche Grenzwerte festgelegt, deren Einhaltung durch Analyse kontrolliert wird. Die Analyse von Pestiziden erfolgt für verschiedene Proben mit unterschiedlicher Probenmatrix. Es ist daher Ziel der Analyse, die Pestizide in den Proben möglichst gut von der Matrix zu trennen und anschließend zu analysieren. Aufgabe der Entwicklung ist neben der Erreichung einer hohen Richtigkeit, die Probenvorbereitung und -analyse zu automatisieren und den Arbeitsaufwand und Verbrauch an Chemikalien für die Analyse gering zu halten.

### Stand der Technik

Die Veröffentlichung "Validation of a Simple and Rapid Multiresidue Method (QuEChERS) and its Implementation in Routine Pesticide Analysis" von M.Anastassiades, E. Scherbaum und D. Bertsch, Poster auf dem MGPR Symposium, Mai 2003 in Aix en Provence, Frankreich offenbart ein vereinfachtes Verfahren zur Probenvorbereitung für die instrumentelle Analyse von Pestiziden mittels GC-MSD oder LC-MS. Das Verfahren ersetzt verschiedene Schritte durch einfachere Schritte. Dabei erfolgt die Zugabe verschiedener Chemikalien, wie MgSO₄, NaCl und Acetonitril und es werden verschiedene Aufbereitungsschritte, wie Schütteln oder Zentrifugieren zur Probenaufbereitung durchgeführt.

Die unter dem Begriff "S19" bekannte, modulare Multimethode zur Bestimmung von Pflanzenschutzmittelrückständen in Lebensmitteln von Specht umfasst im wesentlichen die Extraktion und Verteilung, Gelpermeationschromatographie (GPC), die Chromatographie an einer kleinen Kieselgelsäule und die anschließende gaschromatographische Bestimmung mit verschiedenen Detektoren.

Bei der ChemElut-Methode nach Alder wird die homogenisierte Probe, nach Einstellung eines einheitlichen Wassergehalts, mit Methanol extrahiert und anschließend der zentrifugierte Extrakt durch flüssig-flüssig-Verteilung an Diatomeenerde gereinigt. Dadurch können die zu analysierenden Pestizide von störenden Matrixkomponenten abgetrennt werden. Die zur Elution notwendige relativ große Menge an Dichlormethan wird schließlich eingeengt und der Rückstand in einem zur Messung geeigneten Lösungsmittel, meist Methanol, aufgenommen.

US 2010/024527 A1 (LAMARR WILLIAM A [US] ET AL, 4. Februar 2010) offenbart eine Anordnung und eine Methode zur Probenaufbereitung und Analyse von Proben, enthaltend ein Mehrsäulen-System einen Detektor und eine Ventilanordnung zur Steuerung des Proben- und Matrixstroms.

Bei allen bekannten Verfahren erfolgt eine Automatisierung durch Nachahmung der gewöhnlich von Hand durchgeführten Verfahrensschritte. Dabei erfolgt die Extraktion der Probe mit unterschiedlichen Lösungsmitteln. Die Reinigung und Messung des Extrakts erfordert eine Vielzahl verschiedener Schritte im Labor (flüssig-flüssig Verteilung, SPE, GPC, ...).

Zur Vereinfachung der aufwendigen Probenvobereitung stehen leistungsfähige Detektoren zur Verfügung. Die Automatisierung erfolgt in Anlehnung an den oben aufgeführten Stand der Technik.

Nachteilig bei allen bekannten Verfahren ist der hohe Arbeitsaufwand und der hohe Materialverbrauch. Bei einigen Verfahren sind die Richtigkeit der Ergebnisse und die Anzahl der analysierbaren Pestizide begrenzt.

### Offenbarung der Erfindung

Es ist Aufgabe der Erfindung, die Probenaufbereitung wirtschaftlicher zu gestalten und den Arbeitsaufwand und Materialverbrauch zu senken. Erfindungsgemäß wird die Aufgabe mit einem Verfahren gelöst, mit den Schritten:
(a) Aufgeben einer in einem überwiegend wasserarmen und/oder unpolaren Laufmittel gelösten Probe auf eine HILIC-Chromatographiesäule mit einem überwiegend wasserarmen und/oder unpolaren Laufmittel;
(b) Anreichern zumindest eines großen Teils der in der Probe enthaltenen Pestizide in einer SPE-Anreicherungsanordnung;
(c) Leiten des mit Pestiziden in der SPE-Anreicherungsanordnung angereicherten Probeteils in umgekehrter Richtung von der SPE-Anreicherungsanordnung durch die zweite Chromatographiesäule mit überwiegend wasserreichem und/oder polarem Laufmittel durch Umschalten einer Ventilanordnung nach einer ausgewählten Anreicherungsdauer; und
(d) Detektieren der in der zweiten Chromatographiesäule getrennten Probenbestandteile.

Dabei wird in einer vorteilhaften Ausgestaltung der Erfindung in einem ersten Schaltzustand einer Ventilanordnung das überwiegend wasserreiche und/oder polare Laufmittel mit einer zweiten Pumpe in umgekehrter Richtung durch eine zweite Chromatographiesäule gepumpt und anschließend abgeleitet.

Die erfindungsgemäße Anordnung erlaubt eine vollständige Automatisierung der Probenvorbereitung und Analyse. Die Rohextrakte werden direkt gereinigt, d.h. von störender Matrix befreit, und analysiert. Die Extraktion der Probe vor der Aufgabe erfolgt mit einem wasserarmen und/oder unpolaren Lösungsmittel. Hierzu eignet sich Acetonitril (ACN) besonders gut. Ein Mischungsverhältnis von 95 Vol.-% ACN und 5 Vol.-% Wasser hat sich als besonders vorteilhaft herausgestellt. Das Extrakt kann direkt zur Messung verwendet werden. Die Aufreinigung des Extrakts erfolgt chromatographisch an der HILIC-Chromatographiesäule und automatisiert.

Die Verwendung einer HILIC-Säule ist besonders deshalb vorteilhaft, weil dort die Pestizide von störenden Matrixkomponenten gut getrennt werden können. Die Pestizide eluieren früher als die Matrix und werden in der SPE-Anreicherungsanordnung aufgefangen. Die Matrix bleibt in der HILIC-Chromatographiesäule und kann später eluiert werden.

Man erkennt, dass anders als bei den bisherigen Automatisierungsversuchen hier nicht eine bestehende Methode imitiert wird, sondern ein neues Verfahren angewendet wird. Statt eine flüssig-flüssig Verteilung vorzunehmen, wird eine HILIC-Chromatographiesäule eingesetzt. Während die Pestizide im zweiten Schritt in einer zweiten, analytischen Chromatographiesäule analysiert werden, kann die HILIC-Chromatographiesäule gereinigt werden.

Vorzugsweise wird die zweite, analytische Chromatographiesäule als reverse-phase (RP)-Chromatographiesäule betrieben. Überraschenderweise hat sich herausgestellt, dass die Kopplung einer HILIC-Chromatographiesäule mit einer RP-Chromatographiesäule als Pestizid-Multimethode besonders reproduzierbare Ergebnisse mit guten Nachweisgrenzen und Empfindlichkeiten ergibt.

Die Erfindung erlaubt die Erfassung des Wirkstoffspektrums der bekannten, klassischen Verfahren ohne manuelle Probenvorbereitung. Die Anreicherung in der SPE-Anreicherungsanordnung hat den weiteren Vorteil, dass hohe Injektionsvolumina verwirklicht werden können. Dadurch werden die Nachweisgrenzen und die Empfindlichkeit weiter erhöht.

Die angereicherten Komponenten werden im zweiten Schaltzustand mit beginnendem Gradienten im Backflush-Verfahren auf die zweite, analytische Säule transferiert. Gleichzeitig wird die Matrix von der HILIC-Chromatographiesäule eluiert und die Säule für die nächste Analyse konditioniert. "Wegwerfartikel" werden zur Reinigung nicht eingesetzt. Das schont die Umwelt und macht das Verfahren besonders wirtschaftlich.

Eine bevorzugte Ausgestaltung der Erfindung verwendet als wasserreiches und/oder polares Laufmittel zumindest 90 Vol.-% Wasser zu Beginn der Analyse. Weiterhin kann das wasserreiche und/oder polare Laufmittel 3 bis 7 Vol.-%, vorzugsweise 5 Vol.-% Acetonitril und/oder MeOH enthalten. Mit diesem Laufmittel werden die angereicherten Pestizide von der SPE-Anreicherungsanordnung im Backflush-Verfahren zur zweiten, analytischen Chromatographiesäule geleitet.

Die zweite Chromatographiesäule kann eine High Performance Liquid Column (HPLC)-Säule sein. Es kann aber auch eine Gaschromatographie (GC)-Säule eingesetzt werden. Dann erfolgt die Elution in der SPE-Anreicherungsanordnung offline. Das Eluat wird dann in der GC-Säule analysiert. Mit anderen Worten: die HILIC-Chromatographiesäule und die SPE-Anreicherungsanordnung dienen der Probenvorbereitung für eine GC-Säule. Bei dieser Verwendung wird die angereicherte Probe aus der SPE-Anreicherungsanordnung mit einem Laufmittel, beispielsweise Essigester und/oder Aceton, in ein Gefäß gespült und dann mit der GC-Säule analysiert.

In einer besonders vorteilhaften Ausgestaltung der Erfindung wird ein Teil des Probenstroms, der in einem ersten Schaltzustand während einer ausgewählten Anreicherungszeit durch die SPE-Anreicherungsanordnung hindurch fließt, direkt zum Detektor geleitet. Dies sind insbesondere nicht-anreicherungsfähige, polare Pestizide. Sie werden direkt zum Detektor geleitet und dort gemessen. Gleichzeitig wird die Matrix abgetrennt. Als Detektor ist insbesondere ein Massenspektrometer geeignet.

Ein Teil des Probenstroms, der während einer ausgewählten Anreicherungszeit durch die SPE-Anreicherungsanordnung hindurch fließt, wird dabei vorteilhafterweise direkt detektiert.

Das Verfahren ist besonders vorteilhaft, wenn die HILIC-Chromatographiesäule während eines Teils der Analysenzeit im zweiten Schaltzustand mit der Pumpe am Massenspektrometer regeneriert wird und während der verbleibenden Analysenzeit ein Pestizidprobenteil für die Gaschromatographie mittels der Säule erstellt wird. Dieses hat zur Folge, dass während der LC-MS-Analytik sofort die Probenvorbereitung für die GC-Analytik stattfinden kann.

Ausgestaltungen der Erfindung sind Gegenstand der Unteransprüche. Ein Ausführungsbeispiel ist nachstehend unter Bezugnahme auf die beigefügten Zeichnungen näher erläutert.

### Kurze Beschreibung der Zeichnungen

- Fig.1: ist eine schematische Darstellung einer Anordnung zur Probenvorbereitung und Analyse von Pestiziden in einer ersten Schaltstellung.
- Fig.2: zeigt die Anordnung aus Figur 1 in einer zweiten Schaltstellung.

### Beschreibung des Ausführungsbeispiels

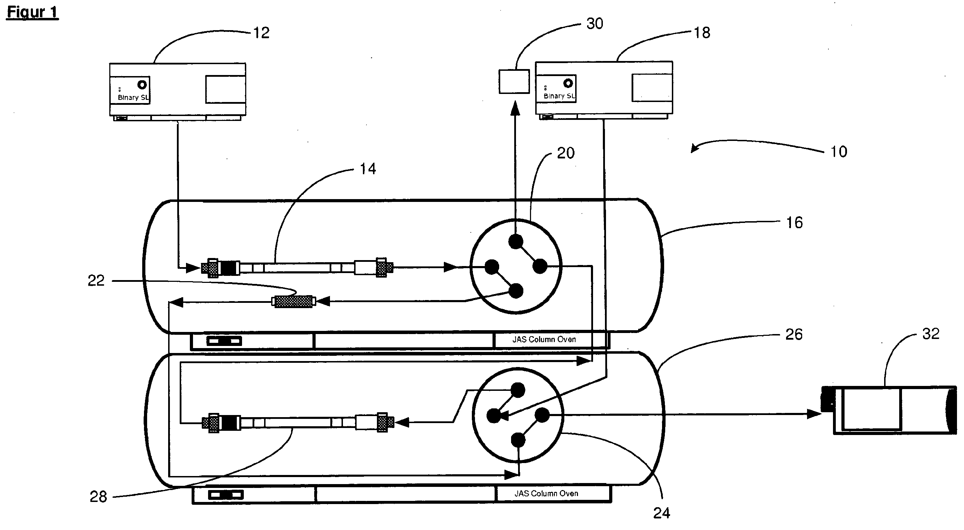

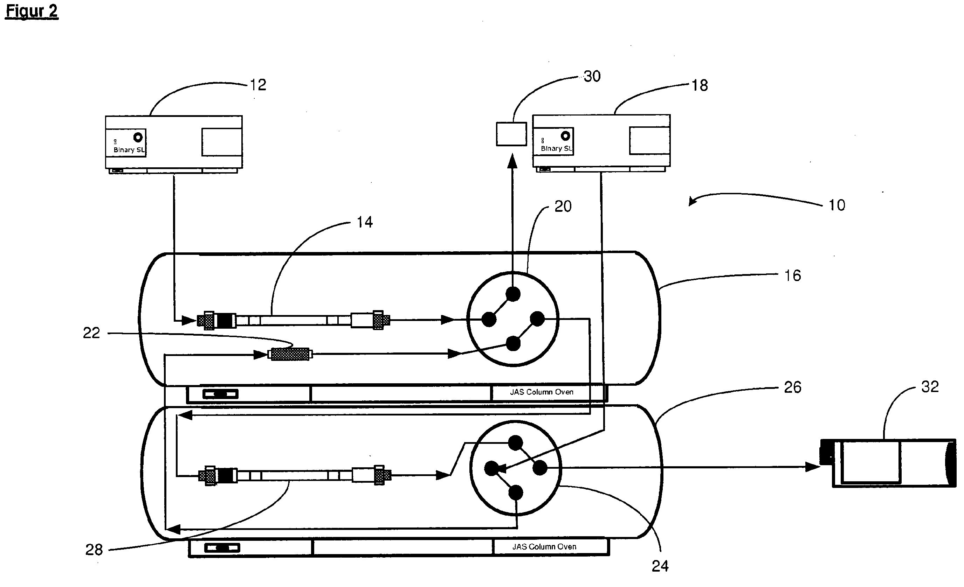

Figuren 1 und 2 zeigt eine Anordnung zur automatischen Analyse von Pestiziden in Proben, die allgemein mit 10 bezeichnet ist. Die Anordnung umfasst eine Gradientenpumpe 12, welche zu Beginn mit einem Laufmittel, bestehend aus 5 Vol.-% Wasser und 95 Vol.-% ACN zu einer HILIC-Chromatographiesäule 14 in einem Ofen 16 pumpt. Die Anordnung umfasst eine weitere Gradientenpumpe 18, welche ein Laufmittel zu Beginn bestehend aus 5 Vol.-ACN und/oder MeOH und 95 Vol.-% Wasser zu einem Ventil 20 pumpt.

In dem in Figur 1 gezeigten Schaltzustand ist der Ausgang der HILIC-Chromatographiesäule 14 über das Ventil 20 mit einer SPE-Anreicherungsanordnung 22 verbunden. Die SPE-Anreicherungsanordnung ist im vorliegenden Ausführungsbeispiel eine kurze C8 Säule. Durch Zumischung von Wasser vor der SPE-Anreicherungssäule (nicht dargestellt) wird die Polarität des Eluats erhöht, so dass die Pestizide angereichert werden können.

Der Ausgang der SPE-Anreicherungsanordnung führt über ein weiteres Ventil 24 in einem Ofen 26 zum Probenraum eines LC-MS-Detektors 32. In dem Ofen 26 ist eine RP-HPLC-Säule (C18-Säule) 28 angeordnet. In dem in Figur 1 gezeigten Schaltzustand führt der Laufmittelstrom aus der Pumpe 18 über das Ventil 24 zur HPLC-Säule 28. Der Ausgang der HPLC-Säule 28 ist über das Ventil 20 mit einer Entsorgung 30 verbunden.

Figur 2 zeigt die gleiche Anordnung wie in Figur 1. Hier sind die Ventile 20 und 24 jedoch anders geschaltet. Das Laufmittel wird mit der Gradientenpumpe 12 durch die HILIC-Säule 14 über das Ventil 20 zur Entsorgung 30 geleitet. Dabei wird die HILIC-Säule gereinigt und so für die nächste Analyse vorbereitet.

Das wasserreiche Laufmittel wird mit der Gradientenpumpe 18 über das Ventil 24 zur SPE-Anreicherungsanordnung 22 geleitet. Dort strömt es in umgekehrter Richtung wie in Figur 1 durch die SPE-Anreicherungsanordnung 22 zum Ventil 20. Das Ventil 20 ist derart geschaltet, dass die Probe mit den Pestiziden über das Ventil 20 zur Analyse in die HPLC-Säule 28 fließt. Der Ausgang der HPLC-Säule 28 ist über das Ventil 24 mit dem LC-MS-Detektor 32 verbunden.

### Die Anordnung arbeitet wie folgt:

Das Verfahren startet mit der Injektion von 5 µl der ungereinigten Extraktlösung. In der ersten Ventilstellung der Ventile 20 und 24 wird die in ACN und Wasser im Volumenverhältnis 95:5 gelöste Probe (Rohextrakt) mit den zu analysierenden Pestiziden von der Pumpe 12 durch die HILIC-Chromatographiesäule 14 geleitet. Dort wird der größte Teil der nicht-interessierenden, störenden Matrix von den Pestiziden getrennt. Die Matrix bleibt überwiegend in der HILIC-Chromatographiesäule 14. Die Pestizide eluieren dabei zum größten Teil alle sehr zeitig etwa nach 1 bis 4 Minuten. Große Teile der Matrix sind zu diesem Zeitpunkt noch auf der HILIC Säule. Der verbleibende Probenanteil fließt zur SPE-Anreicherungsanordnung 22. Dort reichern sich die meisten Pestizide an. Ein kleiner Teil der Pestizide, insbesondere kleinmolekulare, polare Pestizide, bleiben nicht in der SPE-Anreicherungsanordnung 22, sondern fließen während dieser Anreicherungsperiode (Figur 1) durch die SPE-Anreicherungsanordnung 22 hindurch direkt zum Detektor 32. Sie werden somit bereits in dieser Ventilstellung detektiert.

Nach etwa 4 Minuten ist die Matrix in ausreichendem Maße in der HILIC von den zu analysierenden Pestiziden getrennt. Dann werden die Ventile 20 und 24 geschaltet, so dass sich die in Figur 2 dargestellte Situation ergibt. Bei dieser Schaltstellung werden die in der SPE-Anreicherungsanordnung angereicherten Pestizide mit dem wasserreichen Laufmittel aus 95 Vol.-% Wasser und 5 Vol.-% ACN in umgekehrter Richtung von der SPE-Anreicherungsanordnung 22 zur HPLC-Säule 28 geleitet. Gradientenpumpe 2 eluiert die Komponenten von der SPE-Anreicherungsanordnung im Gegenflussprinzip auf die analytische HPLC-Säule. Dort werden die von der Matrix im Wesentlichen befreiten Pestizide getrennt und mit dem Detektor 32 detektiert. Dies geschieht durch den Gradient, der für die Trennung auf der HPLC-Säule 28 verwendet wird. Beginnend mit hohem Wassergehalt wird die Elutionskraft durch Steigerung des Methanol und/oder ACN-Gehalts langsam erhöht. Gleichzeitig wird die HILIC-Chromatographiesäule 14 mit der Gradientenpumpe 12 regeneriert bzw. gereinigt.

In einem nicht-dargestellten, alternativen Ausführungsbeispiel wird eine GC-Analyse vorgenommen, statt die von der Matrix getrennte Probe zu einer HPLC-Säule zu leiten. Dabei wird die Probe von der SPE-Anreicherungsanordnung zunächst in ein separates Gefäß gespült. Die so vorbereitete Probe kann dann in bekannter Weise mittels Gaschromatographie analysiert werden.

## Patentansprüche

1. Verfahren zur Probenaufbereitung und Analyse von Pestiziden in Proben **gekennzeichnet durch** die Schritte:
(a) Aufgeben einer in einem überwiegend wasserarmen und/oder unpolaren Laufmittel gelösten Probe auf eine HILIC-Chromatographiesäule (14) mit einem überwiegend wasserarmen und/oder unpolaren Laufmittel;
(b) Anreichern zumindest eines großen Teils der in der Probe enthaltenen Pestizide in einer SPE-Anreicherungsanordnung (22);
(c) Leiten des mit Pestiziden in der SPE-Anreicherungsanordnung angereicherten Probeteils in umgekehrter Richtung von der SPE-Anreicherungsanordnung **durch** die zweite Chromatographiesäule mit überwiegend wasserreichem und/oder polarem Laufmittel **durch** Umschalten einer Ventilanordnung nach einer ausgewählten Anreicherungsdauer; und
(d) Detektieren der in der zweiten Chromatographiesäule getrennten Probenbestandteile.

2. Verfahren nach Anspruch 1, **dadurch gekennzeichnet, dass** ein Teil des Probenstroms, der während einer ausgewählten Anreicherungszeit durch die SPE-Anreicherungsanordnung (22) hindurch fließt, direkt detektiert wird.

3. Verfahren nach Anspruch 1 oder 2, **dadurch gekennzeichnet, dass** im zweiten Schaltzustand der Ventilanordnung (20, 24) die Pumpe (12) die HILIC-Chromatographiesäule (14) regeneriert und/oder reinigt.

4. Verfahren nach einem der Ansprüche 1 bis 3, **dadurch gekennzeichnet, dass** die HILIC-Chromatographiesäule (14) während eines Teils der Analysenzeit im zweiten Schaltzustand mit der Pumpe (18) am Massenspektrometer (32) regeneriert wird und während der verbleibenden Analysenzeit ein Pestizidprobenteil für die Gaschromatographie mittels der Säule (14) erstellt wird.

5. Verfahren nach einem der vorgehenden Ansprüche, **dadurch gekennzeichnet, dass** das wasserarme und/oder unpolare Laufmittel der Pumpe (12) zu Beginn mindestens 90 Vol.-% Acetonitril (ACN) enthält.

6. Verfahren nach einem der vorgehenden Ansprüche, **dadurch gekennzeichnet, dass** das wasserarme und/oder unpolare Laufmittel der Pumpe (12) zu Beginn 0 bis 10 Vol.-%, vorzugsweise 5 Vol.-% Wasser enthält.

7. Verfahren nach einem der vorgehenden Ansprüche, **dadurch gekennzeichnet, dass** das wasserreiche und/oder polare Laufmittel der Pumpe (18) zumindest 90 Vol.-% Wasser enthält.

8. Verfahren nach einem der vorgehenden Ansprüche, **dadurch gekennzeichnet, dass** das wasserreiche und/oder polare Laufmittel der Pumpe (18) zu 3 bis 10 Vol.-%, vorzugsweise 5 Vol.-% Acetonitril und/oder MeOH enthält.

## Claims

1. A method for sample preparation and analysis of pesticides in samples **characterised by** the steps:
(a) application of a sample, resolved in a solvent which is essentially non-polar and/or has a low water content to a HILIC chromatography column (14) with a solvent which is essentially non-polar and/or has a low water content;
(b) accumulating at least a major portion of the pesticides comprised in the sample in an SPE accumulating assembly (22);
(c) flowing the sample portion with pesticides accumulated in the SPE accumulation assembly in the opposite direction from the SPE accumulation assembly through the second chromatography column with a solvent which is essentially polar and/or has a high water content by switching of a valve assembly after a selected accumulation period; and
(d) detecting the sample portions separated in the second chromatography column.

2. Method according to claim 1, **characterised in that** a portion of the sample flow flowing through the SPE accumulation assembly (22) during a selected accumulation period is directly detected.

3. Method according to claim 1 or 2, **characterised in that** the HILIC chromatography column (14) is regenerated and/or cleaned out in the second switch state of the valve assembly (20, 24) by the pump.

4. Method according to one of the claims 1 to 3, **characterised in that** the HILIC chromatography column (14) is regenerated with the pump (18) at the mass spectrometer with during a portion of the analysis period in the second switching position with the pump (18) of a mass spectrometer (32) and a pesticide sample portion for gas chromatography is generated with the column (14) during the remaining analysis period.

5. Method according to one of the preceding claims, **characterised in that** the solvent which is essentially non-polar and/or has a low water content of the pump (12) initially contains at least 90 Vol.-% acetonitrile (ACN).

6. Method according to one of the preceding claims, **characterised in that** the solvent which is essentially non-polar and/or has a low water content of the pump (12) initially contains 0 to 10 Vol.-%, preferably 5 Vol.-% water.

7. Method according to one of the preceding claims, **characterised in that** the solvent which is essentially polar and/or has a high water content of the pump (18) contains at least 90 Vol.-% water.

8. Method according to one of the preceding claims, **characterised in that** the solvent which is essentially polar and/or has a high water content of the pump (18) contains to 3 to 10 Vol.-%, preferably 5 Vol.-% acetonitrile and/or MeOH.

## Revendications

1. Procédé pour la préparation et l'analyse d'échantillons de pesticides, **caractérisé par** les étapes de :
(a) application d'un échantillon dissous dans un solvant mobile principalement pauvre en eau et/ou apolaire sur une colonne de chromatographie HILIC (14) contenant un solvant mobile principalement pauvre en eau et/ou apolaire ;
(b) enrichissement d'au moins une grande partie des pesticides contenus dans l'échantillon dans une disposition d'enrichissement SPE (22) ;
(c) guidage de l'élément d'échantillon enrichi en pesticides dans la disposition d'enrichissement SPE dans le sens inverse de la disposition d'enrichissement SPE à travers la seconde colonne de chromatographie comprenant un mobile solvant principalement riche en eau et/ou polaire en commutant une disposition de vannes au bout d'une durée d'enrichissement choisie ; et
(d) détection des composants d'échantillon séparés dans la seconde colonne de chromatographie.

2. Procédé selon la revendication 1, **caractérisé en ce qu'**une partie du courant d'échantillon traversant la disposition d'enrichissement SPE (22) pendant un temps d'enrichissement choisi est détectée directement.

3. Procédé selon la revendication 1 ou 2, **caractérisé en ce que** la pompe (12) régénère et/ou nettoie la colonne de chromatographie HILIC (14) dans le second état de commutation de la disposition de vannes (20, 24).

4. Procédé selon l'une des revendications 1 à 3, **caractérisé en ce que** la colonne de chromatographie HILIC (14) est régénérée pendant une partie du temps d'analyse dans le second état de commutation avec la pompe (18) dans le spectromètre de masse (32) et, pendant le temps d'analyse restant, un élément d'échantillon de pesticides est créé pour la chromatographie en phase gazeuse au moyen de la colonne (14) .

5. Procédé selon l'une des revendications précédentes, **caractérisé en ce que** le solvant mobile pauvre en eau et/ou apolaire de la pompe (12) contient, au début, au moins 90% en volume d'acétonitrile (ACN).

6. Procédé selon l'une des revendications précédentes, **caractérisé en ce que** le solvant pauvre en eau et/ou apolaire de la pompe (12) contient, au début, de 0 à 10 % en volume, de préférence 5 % en volume, d'eau.

7. Procédé selon l'une des revendications précédentes, **caractérisé en ce que** le solvant mobile riche en eau et/ou polaire de la pompe (18) contient au moins 90 % en volume d'eau.

8. Procédé selon l'une des revendications précédentes, **caractérisé en ce que** le solvant mobile riche en eau et/ou polaire de la pompe (18) contient de 3 à 10 % en volume, de préférence 5 % en volume, d'acétonitrile et/ou de MeOH.
